(19) Europäisches Patentamt
European Patent Office
Office européen des brevets

(11) **EP 3 928 729 A1**

(12) **DEMANDE DE BREVET EUROPEEN**

(43) Date de publication:
**29.12.2021 Bulletin 2021/52**

(51) Int Cl.:
*A61B 18/02* (2006.01)   *A61F 7/00* (2006.01)
*A61F 7/12* (2006.01)   *A61B 18/00* (2006.01)
*A61B 17/00* (2006.01)

(21) Numéro de dépôt: **21178345.1**

(22) Date de dépôt: **08.06.2021**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **25.06.2020 FR 2006641**

(71) Demandeurs:
• **Commissariat à l'énergie atomique et aux énergies alternatives**
**75015 Paris (FR)**

• **Université Grenoble Alpes**
**38400 Saint-Martin-d'Hères (FR)**
• **Centre Hospitalier Universitaire Grenoble Alpes**
**38700 La Tronche (FR)**

(72) Inventeurs:
• **BORNTRAGER, Quentin**
**73700 Bourg Saint Maurice (FR)**
• **CHABROL, Claude**
**38054 Grenoble cedex 09 (FR)**
• **CHABARDES, Stephan**
**38400 SAINT MARTIN D'HERES (FR)**

(74) Mandataire: **INNOV-GROUP**
**310, avenue Berthelot**
**69372 Lyon Cedex 08 (FR)**

(54) **SONDE IMPLANTABLE DE REFROIDISSEMENT LOCALISÉ**

(57) L'invention concerne une sonde (2) destinée à être implantée, au moins en partie dans un être vivant, pour refroidir de manière localisée au moins une zone de l'être vivant, ladite sonde (2) comportant notamment un organe refroidissant (200) présent à son extrémité distale, destiné à venir en contact avec la zone à refroidir et un module (201) à refroidissement thermoélectrique comportant une zone froide en contact avec l'organe refroidissant (200).

**Fig. 3**

EP 3 928 729 A1

## Description

## Domaine technique de l'invention

La présente invention se rapporte à une sonde destinée à être implantée, au moins en partie dans un être vivant, pour refroidir de manière localisée au moins une zone de l'être vivant.

L'invention concerne également un dispositif de refroidissement intégrant ladite sonde.

## Etat de la technique

Il est connu de refroidir une zone du cerveau en utilisant une sonde disposant à son extrémité d'un doigt refroidissant.

Ces sondes ont notamment pour objectif de réaliser un refroidissement localisé de tissus cibles dans le cerveau en vue d'un traitement de maladies telles que : épilepsie, dépression, TOC, tremblement essentiel, parkinson, dystonies, obésité, maladie de Gilles de la Tourette, ...

Les tissus cibles sont par exemple : le GPI, le STN, le thalamus dans son ensemble, les faisceaux de fibres tels que le MFB ("Médian Forebrain Bunddle"), le ALIC, le CG 25, l'hippocampe, le noyau amygdalien et toute structure ou lésion profonde épileptogène (dysplasie, polymicrogyrie, hétérotopie nodulaire...).

De telles sondes sont notamment décrites dans les brevets US8202308B2 ainsi que dans la demande de brevet US2013/072776A1**.**

Le brevet US8202308B2 propose ainsi une sonde disposant d'un élément de refroidissement disposé à l'extérieur du crâne et d'un dispositif de transport du froid, réalisé sous la forme d'un caloduc, permettant le refroidissement du doigt froid en contact avec le cerveau en transportant les calories vers l'extérieur.

La demande de brevet US2013/072776A1 propose pour sa part une sonde de refroidissement surfacique (corticale) utilisant un module à effet Peltier placé directement à l'extrémité distale de la sonde où se trouve le doigt froid, permettant ainsi de limiter les pertes thermiques entre ce module et le doigt froid. Un dispositif de refroidissement à liquide est employé pour évacuer les calories de l'extrémité distale de la sonde vers l'extérieur du crâne.

Dans ces deux solutions, la dissipation de chaleur n'est pas optimale. Dans le premier document, les calories peuvent être évacuées directement vers l'extérieur car le module de refroidissement est placé en dehors de la boîte crânienne mais cette solution nécessite un transport de chaleur depuis le doigt froid, pouvant occasionner des pertes de puissance calorique, ainsi qu'une très bonne isolation thermique le long de la sonde. Dans le deuxième document, l'évacuation des calories doit se produire de l'extrémité de la sonde vers l'extérieur du corps humain, en utilisant une solution complexe et encombrante, incompatible avec un système implantable long terme. Dans les deux cas, les rendements thermiques ne sont pas optimaux et ces solutions antérieures peuvent s'avérer complexes à mettre en œuvre.

Les demandes de brevets WO92/20289 et WO03005797A1 décrivent également des architectures de sonde à effet refroidissant.

Il existe un besoin de disposer d'une sonde implantable, long terme, en profondeur, permettant de refroidir de manière localisée une zone du cerveau, cette sonde étant optimisée du point de vue thermique et de son rendement énergétique, et minimisant les risques médicaux lors de son utilisation, c'est-à-dire les risques de lésions et/ou d'infections du patient.

## Exposé de l'invention

Ce besoin est comblé par une sonde destinée à être implantée, au moins en partie dans un être vivant, pour refroidir de manière localisée au moins une zone de l'être vivant, ladite sonde présentant une forme allongée suivant un axe longitudinal entre une première extrémité dite proximale et une deuxième extrémité dite distale, ladite extrémité distale étant destinée à venir en contact avec la zone à refroidir, ladite sonde comportant un premier ensemble comprenant :

- Un organe refroidissant présent à son extrémité distale, destiné à venir en contact avec la zone à refroidir,
- Un module à refroidissement thermoélectrique comportant une zone froide en contact avec l'organe refroidissant et une zone chaude,
- Un dispositif de transport de chaleur présentant une première partie en contact avec la zone chaude du module à refroidissement thermoélectrique et une deuxième partie prolongeant la première partie vers l'extrémité proximale de la sonde,
- Une première enveloppe de séparation thermique agencée autour de la première partie dudit dispositif de transfert de chaleur,
- Une deuxième enveloppe dissipatrice de la chaleur agencée au contact de la deuxième partie dudit dispositif de transfert de chaleur, ladite deuxième enveloppe dissipatrice de la chaleur présentant une conductivité thermique supérieure à celle de l'enveloppe de séparation thermique,
- Des moyens de liaison électrique agencés le long de la sonde pour alimenter le module à refroidissement thermoélectrique.

Selon une particularité, le premier ensemble est de type monobloc et présente une configuration rigide, et la sonde comporte un deuxième ensemble réalisé dans un matériau plus souple prolongeant ledit premier ensemble vers son extrémité proximale.

Selon une autre particularité, la sonde comporte des moyens de connexion électrique agencés d'une part sur le premier ensemble et d'autre part sur le deuxième ensemble.

Selon une autre particularité, le deuxième ensemble est réalisé sous la forme d'un tube souple en silicone ou polyuréthane.

Selon une autre particularité, le dispositif de transfert de chaleur est un caloduc ou un système de transfert thermique en graphite pyrolytique.

Selon une autre particularité, la première enveloppe de séparation thermique est fabriquée en silice ou zirconium.

Selon une autre particularité, la deuxième enveloppe dissipatrice de la chaleur est réalisée en saphir ou nitrure d'aluminium.

Selon une autre particularité, le module à refroidissement thermoélectrique est un module à effet Peltier disposant d'une face dite froide et d'une face dite chaude.

Selon une autre particularité, le module à effet Peltier est agencé avec la face froide et la face chaude parallèles audit axe longitudinal.

Selon une autre particularité, le module à effet Peltier est agencé avec la face froide et la face chaude agencées transversalement audit axe longitudinal.

Selon une autre particularité, la sonde comporte une sonde de température agencée en contact avec l'organe refroidissant.

L'invention concerne également un dispositif de refroidissement comprenant une unité de commande et une sonde destinée à être implantée au moins en partie dans un être vivant, pour refroidir de manière localisée au moins une zone de l'être vivant, ladite sonde étant telle que définie ci-dessus.

**Brève description des figures**

D'autres caractéristiques et avantages vont apparaître dans la description détaillée qui suit, faite en regard des dessins annexés dans lesquels :

- La figure 1 représente, de manière schématique, l'architecture du dispositif de refroidissement conforme à l'invention ;
- La figure 2 représente, de manière schématique, le dispositif de refroidissement de l'invention dont la sonde est implantée dans un crâne ;
- La figure 3 représente, de manière schématique, la sonde de l'invention et illustre ses différentes fonctions thermiques ;
- La figure 4 représente, vue en perspective et en transparence, le premier ensemble de la sonde de l'invention ;
- La figure 5A représente, vu en coupe, le dispositif de refroidissement conforme à l'invention ;
- La figure 5B représente, respectivement, les détails A, B et C de la figure 5A ;
- Les figures 6A et 6B montrent deux exemples de réalisation d'un doigt froid situé à l'extrémité distale de la sonde de l'invention ;
- Les figures 7A à 7C montrent des gradients de température pour différentes formes de l'élément refroidissant de la sonde (doigt froid ou refroidissement annulaire) ;
- La figure 8 montre la courbe de variation de la température en périphérie d'un doigt froid à une température de 16°C ;
- Les figures 9A et 9B montrent des gradients de température en périphérie d'un doigt froid, le doigt étant respectivement à une température de 16°C et à une température de 10°C ;
- La figure 10 représente un premier exemple de réalisation d'un module de type Peltier employé dans la sonde de l'invention ;
- La figure 11 représente un deuxième exemple de réalisation d'un module de type Peltier employé dans la sonde de l'invention ;
- Les figures 12A et 12B représentent deux exemples de réalisation du module de type Peltier pouvant être employé dans la sonde de l'invention ;
- La figure 13 montre une variante de réalisation de l'extrémité de la sonde de l'invention ;
- La figure 14 représente une variante de réalisation de l'extrémité distale de la sonde ;
- La figure 15 représente un diagramme montrant la puissance nécessaire au cours du temps pour maintenir le doigt froid de la sonde à une température de 4°C ;
- La figure 16 représente un diagramme montrant la variation de la température moyenne de surface du doigt froid de la sonde au cours du temps pour obtenir un refroidissement de 0.4W ;

**Description détaillée d'au moins un mode de réalisation**

L'objectif de cette invention est de permettre le refroidissement d'un volume de cerveau en profondeur. La solution proposée permet par exemple de refroidir un volume cérébral situé à une distance d'environ 1mm autour du doigt froid à 20°C, température nécessaire pour bloquer une crise d'épilepsie.

Les cibles potentielles sont l'hippocampe, le noyau du thalamus ou toute zone pouvant présenter un foyer épileptogène ou tumoral. Ce dispositif de refroidissement doit être implantable sur du long terme chez l'Homme afin de proposer un traitement des maladies chroniques, des tumeurs, ou toute autre affection pouvant bénéficier des effets d'un traitement thermique.

D'un point de vue thermodynamique, il est connu que tout corps ayant une température supérieure à 0K possède une énergie thermique. On ne peut donc pas créer du froid, seulement transporter de la chaleur (c'est-à-dire des calories) d'une zone vers une autre. Or, ce transfert de chaleur à lieu spontanément du chaud vers le froid et tend à équilibrer les énergies thermiques, et donc les températures. Si l'on souhaite inverser le sens naturel du transfert thermique en transportant des calories d'une zone froide vers une zone chaude (donc refroidir la zone froide et réchauffer la zone chaude), cela implique de payer un coût énergétique de transport en utilisant un système thermodynamique.

Dans le cadre du dispositif de l'invention, il s'agit de refroidir une zone du cerveau en lui enlevant une partie de son énergie thermique. Cela implique de transporter cette énergie et de la relâcher ailleurs.

Le principe de l'invention vise à utiliser la perfusion artérielle du cerveau pour évacuer les calories absorbées sur la zone à refroidir. La sonde de l'invention répond à ce principe, tout en étant adapté à l'anatomie des patients à traiter. Il faut notamment veiller à limiter les risques de lésions mécaniques dans le cerveau, ainsi que la conséquence de potentiels points chauds thermiques.

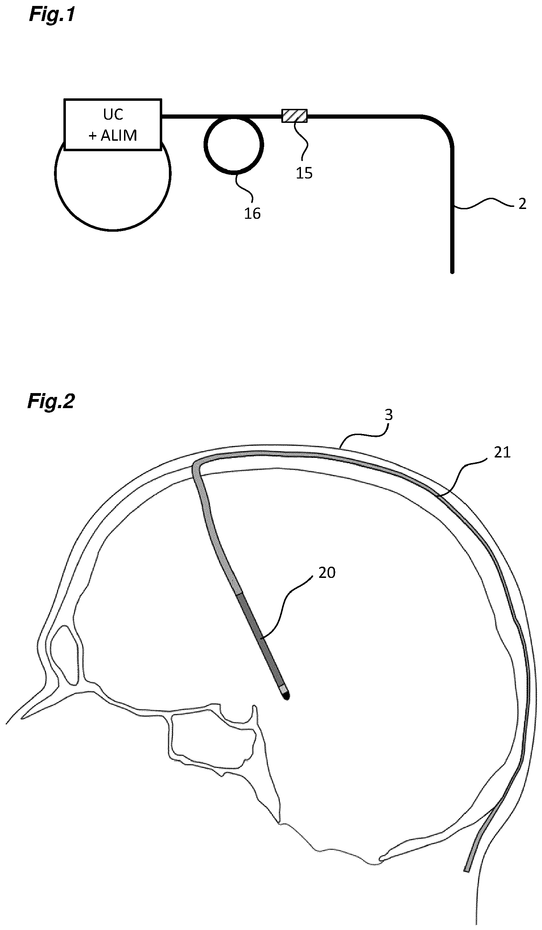

En référence aux figures 1 et 2, le dispositif de refroidissement de l'invention comporte ainsi principalement :

- Une sonde 2 destinée à être implantée au moins en partie dans le corps du patient, à l'intérieur du crâne 3 ;
- Une unité centrale d'alimentation ALIM, et de commande UC avantageusement placée à l'extérieur du crâne du patient, sur laquelle est connectée électriquement la sonde 2 ;

Le dispositif 1 peut comporter des moyens de connexion électrique permettant de connecter la sonde 2 directement à l'unité centrale par son extrémité proximale, ou indirectement par l'intermédiaire d'un connecteur 15 et d'une extension 16.

À l'extrémité distale, la sonde 2 présente avantageusement une forme atraumatique (par exemple avec une forme oblongue ou sphérique - voir ci-après).

De manière générale, la sonde 2 dispose des fonctions et éléments suivants :

- Une partie de la sonde en contact avec le cerveau et permettant le refroidissement ;
- Un système thermodynamique permettant d'inverser le sens naturel du transfert de chaleur ;
- Un dispositif de transport de la chaleur de la zone à refroidir vers une autre zone du cerveau ;
- Un dispositif de séparation thermique entre la partie froide et la partie chaude du dispositif ;
- Un dispositif de répartition thermique permettant d'éviter la création de point chaud ;
- Un dispositif permettant l'alimentation électrique de la sonde en garantissant une liaison mécanique souple à la boîte crânienne ;

Ces différents éléments et fonctions se matérialisent dans la sonde 2 de la manière décrite ci-dessous.

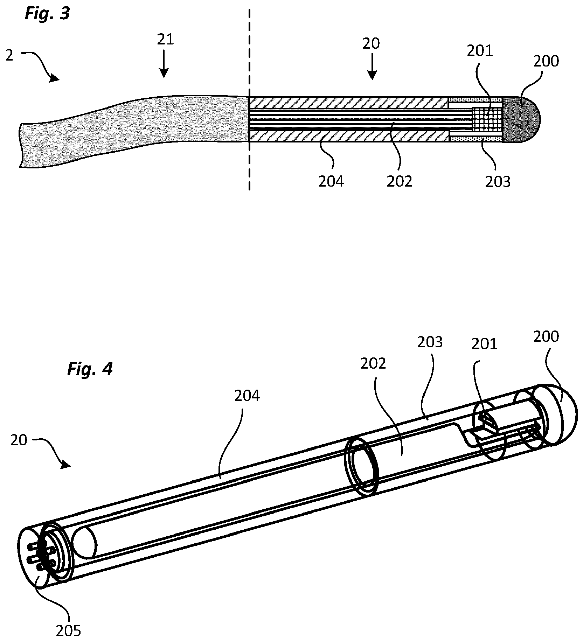

En référence aux figures 2 et 3, la sonde 2 se présente sous la forme d'une tige allongée et développée autour d'un axe longitudinal, dit axe principal, entre une extrémité dite distale portant un organe refroidissant et une extrémité dite proximale par laquelle elle peut être reliée à l'unité de traitement et de commande UC. Elle se compose avantageusement de deux ensembles 20, 21 distincts connectables mécaniquement et électriquement, un premier ensemble 20 rigide qui porte l'organe refroidissant et un deuxième ensemble 21 permettant à la sonde 2 de disposer d'une liaison mécanique souple au niveau de la boîte crânienne. La liaison mécanique à la boîte crânienne doit permettre un maintien de la sonde 2 en place tout en limitant les risques de lésion. À l'intérieur de cette liaison mécanique pourront être intégrés les câbles souples d'alimentation, de contrôle et de mesure de l'implant. Cette liaison disposera avantageusement d'un canal central permettant l'insertion d'un stylet rigide pour faciliter la mise en place chirurgicale. Ce deuxième ensemble peut se présenter sous la forme d'un tube souple, simple canal ou multicanaux, par exemple réalisé en silicone ou en polyuréthane.

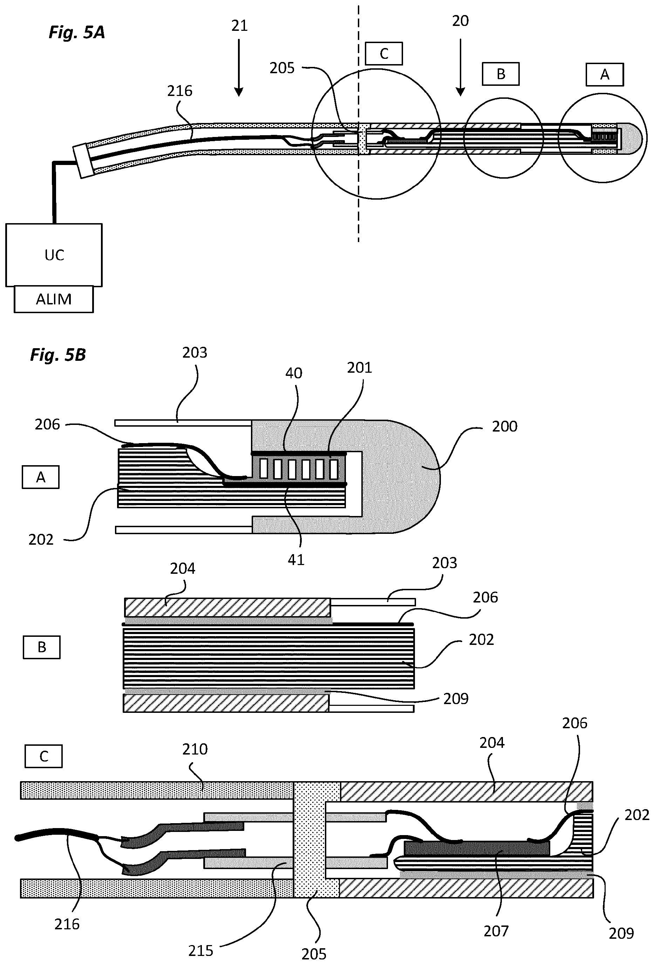

En référence à la figure 4 et aux figures 5A et 5B, le premier ensemble 20 de la sonde 2 est décrit ci-dessous, en allant de son extrémité distale vers son extrémité proximale.

Le premier ensemble 20 de la sonde porte donc à son extrémité distale un organe refroidissant. Cet organe refroidissant est avantageusement un doigt 200 (appelé aussi doigt froid) destiné à être refroidi et à venir en contact avec le tissu cérébral à refroidir. Étant destiné à être en contact avec le cerveau, ce dernier devra être fait dans un matériau biocompatible.

Les dimensions et la forme du doigt 200 refroidissant définissent la zone refroidie ainsi que la puissance nécessaire à ce refroidissement. Avec un doigt froid 200 de taille importante, la zone refroidie sera plus importante, mais les risques médicaux également. Il s'agit donc de définir le compromis optimal.

Le doigt froid 200 doit ainsi répondre aux contraintes suivantes :

- Il doit permettre le refroidissement d'un volume de cerveau le plus important possible sur la zone d'intérêt,
- Il doit disposer d'un encombrement limité,
- Il doit pouvoir être mis en place simplement, évitant tous risques de lésion.

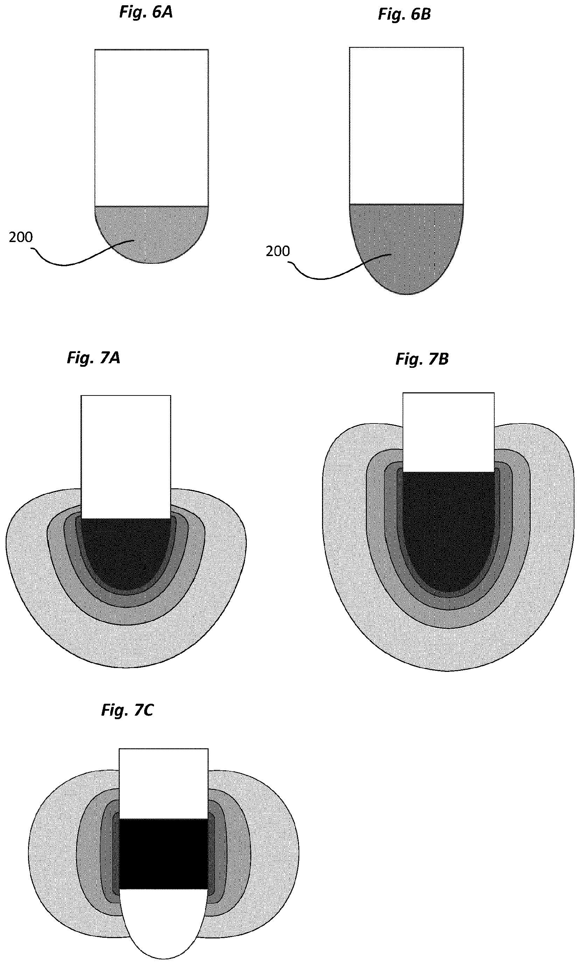

Il doit ainsi disposer d'une surface de contact suffisante et d'une forme finale arrondie ou oblongue, sans angle vif. Les figures 6A et 6B montrent deux exemples de réalisation du doigt froid 200. Il faut noter que l'utilisation d'un oblong plus ou moins prononcé permet de s'adapter au type de cible que l'on souhaite refroidir.

En référence aux figures 7A, 7B et 7C, trois simulations thermiques sont ainsi présentées.

Sur la figure 7A, il s'agit d'une simulation thermique avec une sonde de 3mm de diamètre et un doigt 200 de forme oblongue.

Selon le besoin, afin d'augmenter la surface de contact avec le cerveau, il est possible de prolonger l'hémisphère du doigt froid par une partie cylindrique plus ou moins longue, comme illustré par la figure 7B.

Sur la figure 7C, il s'agit d'une simulation thermique avec une sonde de 3mm de diamètre et un doigt 200 de forme oblongue pour réaliser un refroidissement annulaire.

De manière non limitative, le doigt froid 200 pourra être réalisé dans un matériau tel que le nitrure d'aluminium, silicium, SiC ou du platine iridié, qui sont très bon conducteurs thermiques et biocompatibles. D'autres métaux comme le Titane ou un acier inoxydable peuvent aussi convenir dans une moindre mesure.

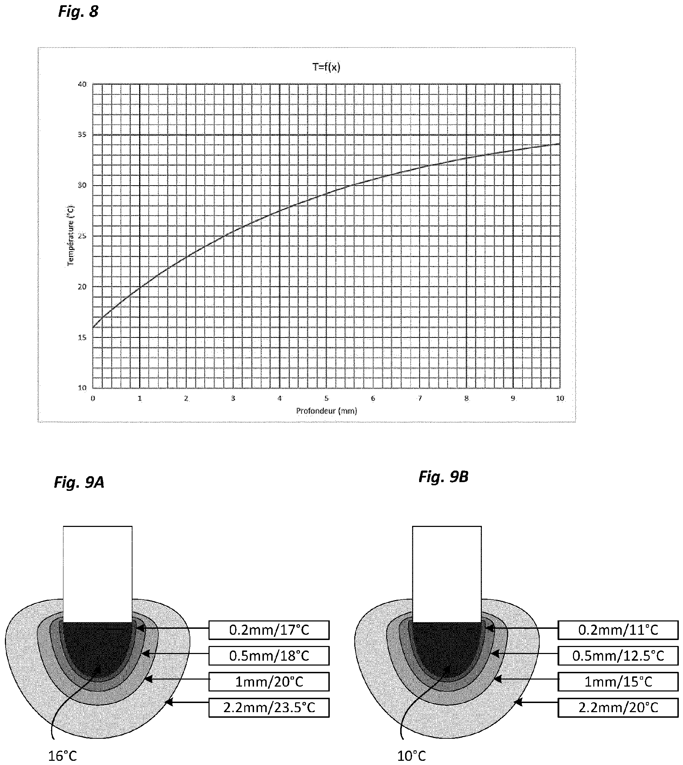

La figure 8 montre une courbe illustrant le gradient de température en périphérie d'un doigt froid ayant une forme telle que celle représentée sur la figure 7A, lorsque celui-ci est à une température de 16°C. La figure 9A illustre ce gradient de température autour du doigt froid à 16°C. La figure 9B montre pour sa part le gradient de température autour de ce même doigt froid lorsque celui-ci est à une température de 10°C.

La sonde 2 comporte un module 201 à refroidissement thermoélectrique. Ce module 201 à refroidissement thermoélectrique peut être un module à effet Peltier, plus précisément de type micro-Peltier pour s'adapter aux dimensions de la sonde. Le module 201 à refroidissement thermoélectrique est destiné à transformer un courant électrique en une différence de température et l'effet Peltier est un effet thermoélectrique consistant en un phénomène physique de déplacement de chaleur en présence d'un courant électrique.

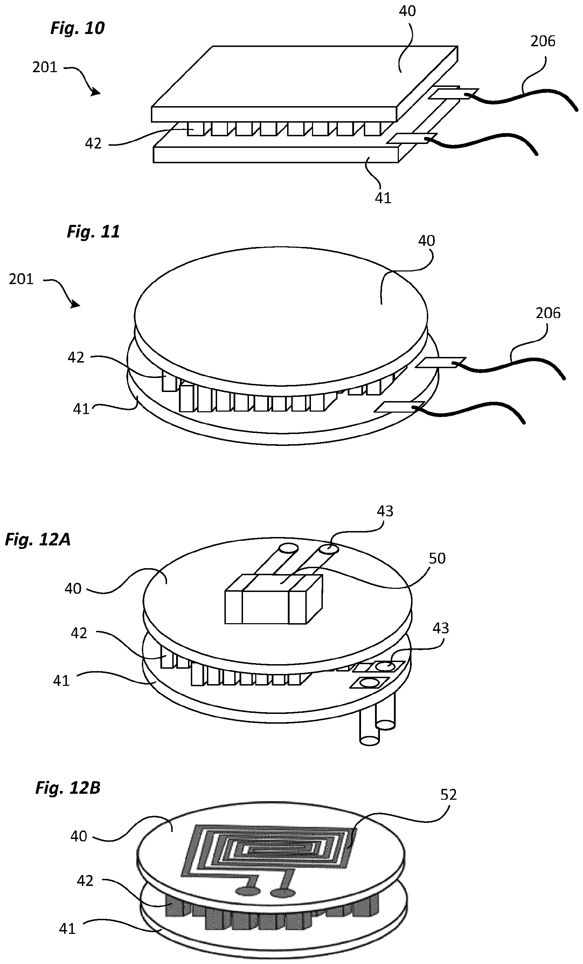

En référence aux figures 10 et 11, un module à refroidissement thermoélectrique est plus précisément une cellule fabriquée avec des composants semiconducteurs 42 asymétriques. Ils sont connectés thermiquement en parallèle et électriquement en série entre une plaque 40 dite froide et une plaque 41 dite chaude. Ces deux plaques 40, 41 sont en général fabriquées en céramique. Les semiconducteurs sont de type P et N. La plaque froide est refroidie par l'absorption d'énergie due au passage des électrons d'un semi-conducteur à l'autre. La plaque chaude 41 récupère l'énergie thermique captée de la plaque froide 40. Il est donc impératif d'évacuer cette chaleur pour qu'elle ne réchauffe pas à nouveau le côté froid.

La sonde 2 ayant un diamètre inférieur ou égal à 3mm, les contraintes dimensionnelles sont élevées. Il est alors primordial de correctement définir le niveau de puissance nécessaire au bon fonctionnement de la sonde. Pour cela, on s'appuie sur les exigences propres à l'utilisation du dispositif :

- Une sonde 2 de diamètre de 1 à 3mm ;
- Une température de doigt froid minimale de 4°C afin de ne pas endommager les tissus ;
- Un doigt froid 200 atteignant, par exemple une température de 16°C en moins de 12 secondes par exemple ;

Après simulations, la puissance de refroidissement de notre système thermodynamique peut être choisie comprise entre 0.1W et 0.4W, en négligeant les pertes. Ces pertes thermiques seront majoritairement liées au contact créé par le packaging entre la zone chaude et la zone froide de notre système.

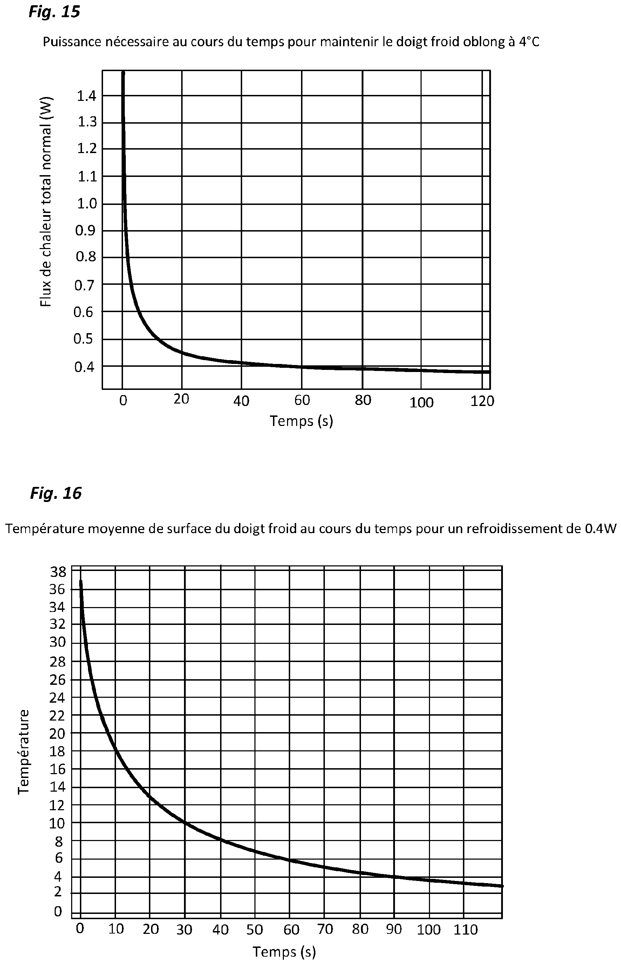

La figure 15 illustre ce choix. Sur ce diagramme, on a tracé, pour une sonde de 3mm de diamètre et un doigt froid oblong en inox de longueur 2.5mm, la puissance nécessaire pour maintenir le doigt à 4°C en fonction du temps.

Pour maintenir le régime stationnaire on aura besoin au minimum d'une puissance de 0.37W de froid, en négligeant les pertes.

Si on effectue une simulation avec le même dispositif et un système thermodynamique d'une puissance de 0.4W on obtient l'évolution de la température du doigt froid au cours du temps après mise en route du système. Cette évolution est représentée sur le diagramme de la figure 16. On peut notamment voir que, avec une puissance de refroidissement de 0.4W, il est possible d'obtenir une température moyenne du doigt froid inférieur à 16°C en moins de 12 secondes.

De manière non limitative, la puissance de refroidissement du système thermodynamique est comprise entre 0.1W et 0.4W, en négligeant les pertes.

De manière non limitative, le module 201 de type micro-Peltier peut être celui proposé par la société TEC MICROSYSTEM sous la référence 1MD02-010-03ANt.

Le module 201 à refroidissement thermoélectrique permet de soutirer des calories au cerveau par l'intermédiaire du doigt froid 200. Pour évacuer les calories et les transporter vers une zone plus éloignée, la sonde 2 comporte ensuite un dispositif 202 de transport de chaleur.

Ce dispositif 202 de transport de la chaleur présente une haute conductivité thermique, par exemple supérieure à 1000 W/m/K.

Cette fonction peut par exemple être réalisée à partir d'un micro-caloduc réalisé dans un matériau de type métallique comme du cuivre ou en silice, saphir ou silicium pour réduire les artéfacts lors d'imagerie IRM.

En variante, il peut également s'agir d'un élément en graphite pyrolytique.

Comme représenté sur la figure 5B, le dispositif 202 de transport de chaleur comporte une première extrémité venant en contact avec la plaque chaude 41 du module 201 à refroidissement thermoélectrique, afin de capter au mieux les calories à évacuer.

De manière non limitative, dans une première variante de réalisation, le dispositif de transport de chaleur peut comporter à cette première extrémité un bec définissant une surface de contact agencé parallèlement à l'axe de la sonde, contre laquelle est apposée la plaque chaude 41 du module 201 à refroidissement thermoélectrique. Le module 201 à refroidissement thermoélectrique présente ainsi sa plaque chaude 41 contre ladite surface de contact et sa plaque froide 40 agencée parallèlement à sa plaque chaude. Le doigt froid 200 peut ainsi comporter une extension lui permettant de se coller sur la plaque froide 40 du module et ainsi assurer le transfert thermique du module vers le doigt froid 200. Le module 201 à refroidissement thermoélectrique présente par exemple la configuration représentée sur la figure 10, avec des plaques 40, 41 de forme rectangulaire.

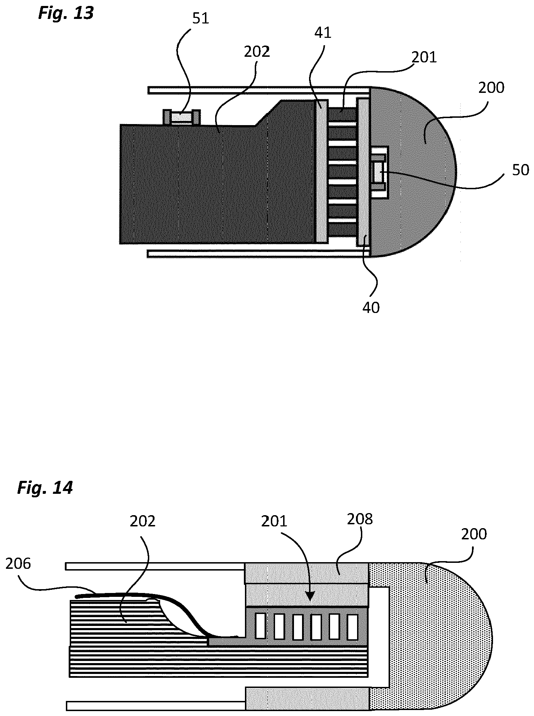

Dans une deuxième variante de réalisation illustrée par la figure 13, la surface de contact peut être agencée perpendiculairement à l'axe de la sonde 2. Le module 201 à refroidissement thermoélectrique présente donc sa plaque chaude 41 agencée transversalement à l'axe de la sonde contre la surface de contact et sa plaque froide 40 parallèle contre laquelle est collé le doigt froid 200. Cette solution permet d'augmenter la puissance disponible et ainsi le gradient thermique.

Dans ce dernier cas, le module 201 à refroidissement thermoélectrique présente alors par exemple la configuration représentée sur la figure 11, avec des plaques 40, 41 en forme de disque, pour s'intégrer transversalement dans la sonde 2.

En référence à la figure 12A, ce module Peltier conçu spécifiquement peut également intégrer des contacts traversants 43 sur les plaques 40, 41 chaudes et froides, afin de faciliter son assemblage dans la sonde 2.

Sur la figure 12A une sonde 50 de température type CMS est présentée sur la face froide 40. Cette sonde de température peut être avantageusement remplacée par une sonde 52 sérigraphiée par exemple PT100, comme représenté sur la figure 12B. Ce type de sonde sérigraphiée peut être réalisé sur les faces externes des plaques 40 et 41.

Le dispositif 202 de transport de chaleur s'étend sur une longueur déterminée non nulle vers le côté proximal de la sonde 2, en suivant l'axe principal de la sonde.

Pour éviter les pertes thermiques à proximité de la zone à refroidir et donc évacuer au mieux les calories grâce au dispositif 202 de transport de chaleur, la sonde 2 comporte un dispositif de séparation thermique. Il s'agit avantageusement d'une enveloppe de séparation thermique, dite enveloppe isolante 203, agencée autour d'une première partie de la longueur du dispositif 202 de transport de chaleur. Le rôle de cette enveloppe isolante 203 est de séparer thermiquement la partie doigt froid et la partie répartiteur thermique. Elle ne dispose d'aucun contact thermique avec le caloduc ou le module Peltier (voir ci-après). Comme il s'agit de refroidir une zone du cerveau, tout apport de chaleur dans cette zone constitue en effet une diminution de l'efficacité du système et il est donc essentiel de bien séparer la zone froide de la zone chaude. Par ailleurs, la longueur de l'enveloppe isolante 203 doit être la plus faible possible afin de consacrer une longueur maximale à la répartition thermique. La puissance de refroidissement du module 201 à refroidissement thermoélectrique étant comprise entre 100W et 400mW, l'enveloppe isolante 203 pourra s'étendre sur une longueur d'au moins 6mm et avec une épaisseur la plus faible possible, par exemple 0.25mm. Ces données dimensionnelles sont à considérer de manière non limitative et pourront bien entendu varier selon la puissance de refroidissement du module 201 à refroidissement thermoélectrique.

De manière non limitative, cette enveloppe isolante 203 peut se présenter sous la forme d'une bague en silice ou en zirconium.

L'ensemble des modules 200, 203, 204 et 205 est assemblé hermétiquement par brasure ou soudure laser.

Selon l'invention, sur une deuxième partie de la longueur du dispositif 202 de transport de chaleur, la sonde 2 comporte un dispositif de répartition thermique (appelé également ci-dessus "répartiteur" de chaleur). Ce dispositif est destiné à permettre la libération des calories transportées par le dispositif de transport de chaleur à une distance suffisante de la zone à refroidir, en utilisant la perfusion artérielle du cerveau. Ce dispositif agit ainsi comme un échangeur thermique en vue d'évacuer les calories absorbées au niveau du doigt froid. Son objectif est donc de céder de l'énergie thermique au cerveau environnant, sans créer de lésion.

En référence aux figures 4 et 5B, ce dispositif de répartition thermique se présente sous la forme d'une enveloppe 204 à bonne conductivité thermique, dite enveloppe conductrice 204, agencée autour d'une deuxième partie du dispositif 202 de transport de chaleur. Cette enveloppe 204 conductrice prolonge l'enveloppe isolante 203 réalisant la séparation thermique, en direction de son extrémité proximale. Elle présente donc bien entendu une conductivité thermique supérieure à celle de l'enveloppe isolante 203. Elle vient avantageusement au contact de la deuxième partie du dispositif 202 de transport de chaleur. A titre d'exemple, elle peut être assemblée à celui-ci par collage conducteur thermique 209.

En ce qui concerne la longueur optimale de cette enveloppe conductrice 204, on peut s'appuyer sur l'équation de transfert thermique d'un échangeur thermique :

$$P = h \times S \times DT$$

Avec

- P la puissance à céder au cerveau, correspondant à la puissance soustraite au doigt froid divisée par le rendement du module à refroidissement thermoélectrique ;
- h le coefficient d'échange total ;
- S la surface de l'interface échangeur-cerveau ;
- DT la différence de température entre l'échangeur et le cerveau ;

La seule variable sur laquelle il est possible d'agir est la surface de l'échangeur. Le diamètre de la sonde 2 étant fixé, on cherche la longueur optimale de l'échangeur (l'enveloppe conductrice) pour éviter une surchauffe du cerveau, pouvant entraîner des lésions. On choisit par exemple de se limiter à un échauffement maximal du cerveau de 2°C par rapport à l'équilibre.

De manière non limitative, il est possible de disposer d'une longueur au moins égale à 50mm d'échangeur pour un diamètre 2.5 à 3mm, ce qui permet d'éviter une surchauffe au bout de 3 minutes de fonctionnement avec une puissance de 0.35W.

De manière non limitative, cette enveloppe conductrice 204 peut être réalisée dans un matériau tel qu'un métal, titane, permettant d'éviter la création de point chaud. Elle peut également être réalisée dans un matériau de type saphir, nitrure d'aluminium ou alumine pour améliorer la compatibilité IRM. Pour réaliser un contact thermique entre 202 et 204, une résine (209) époxy, polyuréthane ou silicone chargée (pour améliorer sa conductivité thermique) peut par exemple être injectée dans le canal interne de la sonde 2, avantageusement uniquement autour de la deuxième partie du dispositif 202 de transport de chaleur 202, pour combler l'espace entre celui-ci et l'enveloppe conductrice 204.

A son extrémité proximale, le premier ensemble 20 de la sonde 2 peut proposer une traversée électrique 205 sur laquelle sont réunis les conducteurs d'alimentation du module 201 à refroidissement thermoélectrique ainsi que d'autres points de connexion permettant le transfert de données (température, électrodes de mesure). Elle permet de raccorder le premier ensemble 20 de la sonde au deuxième ensemble 21 de la sonde.

Les conducteurs 206 peuvent être des pistes électriques d'un circuit électrique flexible, par exemple réalisé en polyimide, ou agencées directement sur le dispositif 202 de transport de chaleur. Une carte 207 de reprise de contact peut être agencée pour supporter tous composants électriques de pilotage, conditionnement de signaux, de sécurité électrique et connecter les conducteurs aux broches respectives de la traversée électrique. Bien entendu, les solutions de liaison et de connexion électrique ne faisant pas l'objet de l'invention, toute autre solution pourrait être envisagée.

L'enveloppe externe du dispositif, formée par celle du doigt froid 200, de l'enveloppe isolante 203, de l'enveloppe conductrice 204 et la traversée électrique 205 respectent les conditions de biocompatibilité et sont assemblables entre elles, par exemple à l'aide de technologies telles que brasure sous vide et/ou laser.

Le deuxième ensemble 21 de la sonde peut se présenter sous la forme d'un tube 210 souple mono canal ou multicanal, par exemple en silicone ou polyuréthane biocompatible. Les conducteurs 206 se prolongent à l'intérieur du tube par un câble 216 souple pour rejoindre l'unité de traitement et de commande UC. Les câbles 216 peuvent être raccordés aux contacts 215 par soudure par point ou soudure laser avant assemblage du tube de protection 210 par collage.

De manière non limitative, l'unité de commande UC comporte au moins un microprocesseur et des moyens de mémorisation. Elle est destinée à exécuter des instructions logicielles représentatives d'une séquence de traitement de la pathologie par le dispositif. Elle comporte notamment des moyens de commande du dispositif de refroidissement. Elle comporte également une ou plusieurs interfaces de communication destinées à communiquer avec les différentes entités, notamment des moyens de détection. Les liaisons de communication pourront être filaire ou sans-fil.

Le dispositif de refroidissement comporte une source d'alimentation électrique ALIM, pour alimenter la sonde et les autres éléments du dispositif. De manière non limitative, la source d'alimentation électrique ALIM peut être une batterie rechargeable. La batterie et l'unité de commande UC sont avantageusement intégrés dans un boitier hermétique équipé d'un connecteur et situés à l'extérieur du crâne 3.

Différents compléments peuvent être apportés à la sonde de l'invention. Nous donnons ci-dessous quelques exemples de solutions complémentaires qu'il est possible d'intégrer à cette sonde.

On peut intégrer une solution de surveillance de la température en différents points de la sonde. La figure 13 donne quelques exemples de placement de capteurs de température sur la sonde. Cela s'applique bien entendu aux différentes variantes de sonde 2, à module 201 de refroidissement thermoélectrique axial (figure 5A) ou transversal (figure 13).

On peut ainsi placer une première sonde 50 (par exemple thermistance) au niveau du doigt froid, sur la plaque froide 40 du module à refroidissement thermoélectrique. Une deuxième sonde 51 (par exemple thermistance) peut être placée au niveau de la zone chaude en contact avec la plaque 41, et une autre au niveau de la zone d'échange thermique de l'enveloppe conductrice 204. Il faut noter que ces sondes peuvent également être réalisées sous la forme d'une sérigraphie 52, comme représenté sur la figure 12B.

Un relais thermostatique peut également être employé dans la partie chaude 204, idéalement sur la carte électronique 207 pour couper l'alimentation en cas de défaillance du système de régulation et garantir ainsi la sécurité du patient. La température de la partie froide 200 est sécurisée par la limitation en puissance du système.

Les données de températures mesurées par les différents capteurs sont avantageusement envoyées vers l'unité de commande UC en vue de contrôler en temps réel le niveau de refroidissement appliqué par le dispositif de l'invention et de le réguler, si nécessaire, en exécutant une boucle de régulation de température. Les capteurs de température peuvent être tous types de capteurs de température (thermistance, PT100, thermocouples), connecté via une liaison filaire à ladite unité de commande UC. En cas de liaison filaire, le fil peut traverser la sonde 2 suivant une direction parallèle à son axe.

Des moyens de détection peuvent être intégrés en parallèle pour détecter l'apparition de la pathologie à traiter. Dans le cas d'une crise d'épilepsie, ces moyens de détection sont implantés au niveau crânien pour détecter une zone ciblée. Lorsque les prémices d'une crise sont détectées, les moyens de détection peuvent envoyer un signal à l'unité de commande UC. L'unité de commande UC alimente la sonde 2. Le dispositif de refroidissement de l'invention est commandé pour générer un refroidissement adapté à la pathologie traitée. Les moyens de détection peuvent comporter des électrodes de type EEG ou des électrodes de types SEEG (stéréo-électro-encéphalographie intracrânienne). Elles peuvent aussi être réalisées par assemblage de bagues métallique ou dépôt localisé directement sur le doigt froid pour être au plus proche de la zone à traiter.

Une régulation de température peut être opérée grâce au capteur de température placé en bout de sonde, en fonction d'une température de consigne. La température de consigne peut être programmée dans l'unité de commande UC. S'il s'agit d'une crise d'épilepsie, l'intensité du refroidissement et sa durée d'application seront préférentiellement liés au niveau d'intensité de la crise qui a été mesuré par les moyens de détection. Dans ce cadre, étant donné qu'un léger échauffement apparaît lors d'une crise d'épilepsie, les différents capteurs de température proposés ci-dessus pourraient permettre d'éliminer les faux positifs lors d'une détection réalisée à l'aide des électrodes SEEG et ainsi renforcer le principe de détection.

L'unité de commande UC du dispositif peut intégrer un redresseur de courant de manière à limiter les risques inhérents aux courants de fuite et ainsi permettre une alimentation en courant alternatif sinusoïdal ou pulsé générant un courant moyen nul.

L'implant peut être mis en place à l'aide d'un guide pelable ou d'un mandrin rigide. Ces derniers devront être retirés une fois le dispositif en place.

En référence à la figure 14, il est également envisageable de réaliser le doigt 200 situé à l'extrémité de la sonde dans un matériau non conducteur thermique, par exemple en silice ou en zirconium et d'insérer une bague 208 refroidissante, directement autour du module 201 à refroidissement thermoélectrique. Le refroidissement n'est ainsi plus réalisé de manière axiale mais de manière radiale. Les autres particularités de la sonde 2 sont conservées. La figure 7C déjà évoquée ci-dessus illustre par ailleurs le gradient de température obtenu autour de cette bague refroidissante.

La solution de l'invention présente ainsi de nombreux avantages, parmi lesquels :

- Une architecture thermique optimisée, en ce qui concerne l'apport du froid en bout de sonde et l'évacuation des calories ;
- Une sonde de taille et de forme adaptées à l'implantation long terme, permettant d'éviter les lésions mécaniques, tout en garantissant une robustesse suffisante et en permettant de refroidir la zone ciblée de manière efficace, avec un rendement optimal ;
- Une solution qui dispose de propriétés mécaniques adaptées à une implantation long terme et en profondeur, notamment grâce à sa structure en deux parties (ensemble rigide et ensemble souple) ;
- Une solution qui utilise des matériaux adaptés à une implantation long terme et en profondeur, tous biocompatibles ;

**Revendications**

1. Sonde (2) destinée à être implantée, au moins en partie dans un être vivant, pour refroidir de manière localisée au moins une zone de l'être vivant, ladite sonde (2) présentant une forme allongée suivant un axe longitudinal entre une première extrémité dite proximale et une deuxième extrémité dite distale, ladite extrémité distale étant destinée à venir en contact avec la zone à refroidir, ladite sonde (2) étant **caractérisée en ce qu'**elle comporte un premier ensemble (20) comprenant :

   - Un organe refroidissant (200) présent à son extrémité distale, destiné à venir en contact avec la zone à refroidir,
   - Un module (201) à refroidissement thermoélectrique comportant une zone froide en contact avec l'organe refroidissant (200) et une zone chaude,
   - Un dispositif (202) de transport de chaleur présentant une première partie en contact avec la zone chaude du module (201) à refroidissement thermoélectrique et une deuxième partie prolongeant la première partie vers l'extrémité proximale de la sonde,
   - Une première enveloppe (203) de séparation thermique agencée autour de la première partie dudit dispositif (202) de transfert de chaleur,
   - Une deuxième enveloppe (204) dissipatrice de la chaleur agencée au contact de la deuxième partie dudit dispositif (202) de transfert de chaleur, ladite deuxième enveloppe (204) dissipatrice de la chaleur présentant une conductivité thermique supérieure à celle de l'enveloppe de séparation thermique,
   - Des moyens de liaison électrique agencés le long de la sonde pour alimenter le module à refroidissement thermoélectrique.

2. Sonde selon la revendication 1, **caractérisée en ce que** le premier ensemble (20) est de type monobloc et présente une configuration rigide, et **en ce que** la sonde comporte un deuxième ensemble (21) réalisé dans un matériau plus souple prolongeant ledit premier ensemble vers son extrémité proximale.

3. Sonde selon la revendication 2, **caractérisée en ce**

**qu'**elle comporte des moyens (205) de connexion électrique agencés d'une part sur le premier ensemble (20) et d'autre part sur le deuxième ensemble (21).

4. Sonde selon la revendication 2 ou 3, **caractérisée en ce que** le deuxième ensemble (21) est réalisé sous la forme d'un tube souple en silicone ou polyuréthane.

5. Sonde selon l'une des revendications 1 à 4, **caractérisée en ce que** le dispositif (202) de transfert de chaleur est un caloduc ou un système de transfert thermique en graphite pyrolytique.

6. Sonde selon l'une des revendications 1 à 5, **caractérisée en ce que** la première enveloppe (203) de séparation thermique est fabriquée en silice ou zirconium.

7. Sonde selon l'une des revendications 1 à 6, **caractérisée en ce que** la deuxième enveloppe (204) dissipatrice de la chaleur est réalisée en saphir ou nitrure d'aluminium.

8. Sonde selon l'une des revendications 1 à 7, **caractérisée en ce que** le module (201) à refroidissement thermoélectrique est un module à effet Peltier disposant d'une face dite froide et d'une face dite chaude.

9. Sonde selon la revendication 8, **caractérisée en ce que** le module à effet Peltier est agencé avec la face froide et la face chaude parallèles audit axe longitudinal.

10. Sonde selon la revendication 8, **caractérisée en ce que** le module à effet Peltier est agencé avec la face froide et la face chaude agencées transversalement audit axe longitudinal.

11. Sonde selon l'une des revendications 1 à 10, **caractérisée en ce qu'**elle comporte une sonde de température (50) agencée en contact avec l'organe refroidissant.

12. Dispositif de refroidissement comprenant une unité de commande (UC) et une sonde (2) destinée à être implantée au moins en partie dans un être vivant, pour refroidir de manière localisée au moins une zone de l'être vivant, **caractérisé en ce que** ladite sonde (2) est telle que définie dans l'une des revendications 1 à 11.

**Fig.1**

**Fig.2**

*Fig. 3*

*Fig. 4*

Fig. 5A

Fig. 5B

**Fig. 6A**

**Fig. 6B**

200

200

**Fig. 7A**

**Fig. 7B**

**Fig. 7C**

Fig. 8

T=f(x)

Fig. 9A

Fig. 9B

0.2mm/17°C
0.5mm/18°C
1mm/20°C
2.2mm/23.5°C

16°C

0.2mm/11°C
0.5mm/12.5°C
1mm/15°C
2.2mm/20°C

10°C

**Fig. 10**

**Fig. 11**

**Fig. 12A**

**Fig. 12B**

**Fig. 13**

**Fig. 14**

*Fig. 15*

Puissance nécessaire au cours du temps pour maintenir le doigt froid oblong à 4°C

*Fig. 16*

Température moyenne de surface du doigt froid au cours du temps pour un refroidissement de 0.4W

Europäisches
Patentamt

European
Patent Office

Office européen
des brevets

**RAPPORT DE RECHERCHE EUROPEENNE**

Numéro de la demande

EP 21 17 8345

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 92/20289 A1 (HAMILTON ARCHIE C [US]) 26 novembre 1992 (1992-11-26) | 1,5,8-12 | INV. A61B18/02 |
| Y | * page 1, ligne 11 - ligne 13 * * page 10, ligne 8 - ligne 17 * * page 12, ligne 11 - ligne 13 * * figure 2 * | 2-4,6,7 | A61F7/00 A61F7/12 |
| | ----- | | ADD. A61B18/00 |
| X | WO 03/005797 A2 (AD TECH MEDICAL INSTR CORP [US]; PUTZ DAVID A [US]; ZIOBRO JOHN [US]) 23 janvier 2003 (2003-01-23) | 1,12 | A61B17/00 |
| A | * page 6, ligne 1 - ligne 3 * * page 15, ligne 8 - ligne 13 * * figures 1A,2 * | 2-11 | |
| | ----- | | |
| Y | FR 3 055 796 A1 (COMMISSARIAT ENERGIE ATOMIQUE [FR]) 16 mars 2018 (2018-03-16) | 2-4 | |
| A | * page 7, ligne 6 - ligne 7 * | 1,5-12 | |
| | ----- | | |
| Y | FR 3 075 034 A1 (COMMISSARIAT ENERGIE ATOMIQUE [FR]) 21 juin 2019 (2019-06-21) | 6 | |
| A | * page 7, ligne 5 - ligne 6 * | 1-5,7-12 | DOMAINES TECHNIQUES RECHERCHES (IPC) |
| | ----- | | |
| Y | US 2006/167445 A1 (SHAFIRSTEIN GAL [US]) 27 juillet 2006 (2006-07-27) | 7 | A61B A61F |
| A | * alinéa [0054] * | 1-6,8-12 | |
| | ----- | | |
| A | WO 89/04137 A1 (ANAND RAJ K [US]) 18 mai 1989 (1989-05-18) * figures 1,5 * | 1-12 | |
| | ----- | | |
| A | WO 2018/217147 A1 (NEURONANO AB [SE]) 29 novembre 2018 (2018-11-29) * figures 1-10 * | 1-12 | |
| | ----- | | |

Le présent rapport a été établi pour toutes les revendications

| Lieu de la recherche | Date d'achèvement de la recherche | Examinateur |
|---|---|---|
| La Haye | 28 octobre 2021 | Milles, Julien |

**ANNEXE AU RAPPORT DE RECHERCHE EUROPEENNE
RELATIF A LA DEMANDE DE BREVET EUROPEEN NO.**

EP 21 17 8345

28-10-2021

| Document brevet cité au rapport de recherche | | Date de publication | Membre(s) de la famille de brevet(s) | | Date de publication |
|---|---|---|---|---|---|
| WO 9220289 | A1 | 26-11-1992 | AU | 2224992 A | 30-12-1992 |
| | | | US | 5207674 A | 04-05-1993 |
| | | | WO | 9220289 A1 | 26-11-1992 |
| WO 03005797 | A2 | 23-01-2003 | AU | 2002320549 A1 | 29-01-2003 |
| | | | CA | 2453673 A1 | 23-01-2003 |
| | | | EP | 1407090 A2 | 14-04-2004 |
| | | | US | 2003014097 A1 | 16-01-2003 |
| | | | WO | 03005797 A2 | 23-01-2003 |
| FR 3055796 | A1 | 16-03-2018 | EP | 3512473 A1 | 24-07-2019 |
| | | | FR | 3055796 A1 | 16-03-2018 |
| | | | US | 2021282963 A1 | 16-09-2021 |
| | | | WO | 2018051005 A1 | 22-03-2018 |
| FR 3075034 | A1 | 21-06-2019 | AUCUN | | |
| US 2006167445 | A1 | 27-07-2006 | US | 2006167445 A1 | 27-07-2006 |
| | | | US | 2008229715 A1 | 25-09-2008 |
| | | | US | 2010318077 A1 | 16-12-2010 |
| | | | US | 2014378956 A1 | 25-12-2014 |
| | | | WO | 2007106263 A2 | 20-09-2007 |
| WO 8904137 | A1 | 18-05-1989 | AU | 2722588 A | 01-06-1989 |
| | | | CA | 1306162 C | 11-08-1992 |
| | | | DE | 3853480 T2 | 04-01-1996 |
| | | | EP | 0386111 A1 | 12-09-1990 |
| | | | JP | H03502532 A | 13-06-1991 |
| | | | US | 4860744 A | 29-08-1989 |
| | | | WO | 8904137 A1 | 18-05-1989 |
| WO 2018217147 | A1 | 29-11-2018 | AU | 2018273484 A1 | 28-11-2019 |
| | | | CN | 110678222 A | 10-01-2020 |
| | | | EP | 3630269 A1 | 08-04-2020 |
| | | | JP | 2020520743 A | 16-07-2020 |
| | | | KR | 20200010309 A | 30-01-2020 |
| | | | US | 2020093512 A1 | 26-03-2020 |
| | | | WO | 2018217147 A1 | 29-11-2018 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8202308 B2
- US 2013072776 A1
- WO 9220289 A
- WO 03005797 A1